# EUROPEAN PATENT SPECIFICATION

(11) **EP 1 988 173 B1**
(45) Date of publication and mention of the grant of the patent: **05.08.2009**
(21) Application number: 08290365.9
(22) Date of filing: 14.04.2008
(51) Int. Cl.: C12Q 1/04, C12Q 1/06

(54) **New composition for permeabilizing the walls of microorganisms comprising the combination of ethylenediaminetetraacetic acid (EDTA) and polyethyleneimine (PEI)**
Neue Zusammensetzung zur Permeabilisierung der Wände von Mikroorganismen mit der Kombination von Ethylendiamintetraessigsäure (EDTA) und Polyethylenimin (PEI)
Nouvelle composition pour la perméabilisation des parois de micro-organismes comprenant une combinaison d'acide éthylènediaminetétraacétique (EDTA) et de polyéthylèneimine (PEI)

(30) Priority: 20.04.2007 FR 0754623
(43) Date of publication of application: 05.11.2008
(73) Proprietor: Millipore Corporation, Billerica, MA 01821 (US)
(72) Inventor: Marc, Frédéric, 67140 Itterswiller (FR); Hohnadel, Marisa, 67000 Strasbourg (FR)
(74) Representative: Caen, Thierry Alain

(56) References cited:
- US-A1- 2006 134 729
- ALAKOMI H-L ET AL: "Weakening effect of cell permeabilizers on gram-negative bacteria causing biodeterioration." APPLIED AND ENVIRONMENTAL MICROBIOLOGY JUL 2006, vol. 72, no. 7, July 2006 (2006-07), pages 4695-4703, XP002462993 ISSN: 0099-2240
- HELANDER I M ET AL: "Polyethyleneimine is an effective permeabilizer of gram-negative bacteria" MICROBIOLOGY, SOCIETY FOR GENERAL MICROBIOLOGY, READING, GB, vol. 143, no. PART 10, October 1997 (1997-10), pages 3193-3199, XP002394264 ISSN: 1350-0872
- NIKAIDO H: "Preventing drug access to targets: Cell surface permeability barriers and active efflux in bacteria" SEMINARS IN CELL AND DEVELOPMENTAL BIOLOGY 2001 GB, vol. 12, no. 3, 2001, pages 215-223, XP002499670 ISSN: 1084-9521
- CANOVAS M ET AL: "Permeabilization of Escherichia coli cells in the biotransformation of trimethylammonium compounds into l-carnitine" ENZYME AND MICROBIAL TECHNOLOGY, STONEHAM, MA, US, vol. 37, no. 3, 2 August 2005 (2005-08-02), pages 300-308, XP004965972 ISSN: 0141-0229

### SEQUENCE LISTING

<110> MILLIPORE CORPORATION
<120> New composition for permeabilizing the walls of microorganisms comprising the combination ofethylenediaminetetraacetic acid (EDTA) and polyethyleneimine (PEI)
<130> BIF117190FR
<150> FR 0754623
   <151> 2007-04-20
<160> 5
<170> PatentIn version 3.1
<210> 1
   <211> 25
   <212> DNA
   <213> artificial DNA
<220>
   <221> misc_binding
   <222> (1)..(25)
   <223> hybridization probe for the specific detection of Pseudomonas aeruginosa RNA
<400>
   1 tctaccgcgt cacttacgtg acacc 25
<210> 2
   <211> 25
   <212> DNA
   <213> artificial DNA
<220>
   <221> misc_binding
   <222> (1)..(25)
   <223> hybridization probe for the specific detection of Bacillus subtilis RNA
<400>
   2 tttgtgggat tggcttaacc tcgcg 25
<210> 3
   <211> 22
   <212> DNA
   <213> artificial DNA
<220>
   <221> misc_binding
   <222> (1)..(22)
   <223> hybridization probe for the specific detection of Staphylococcus aureus RNA
<400> 3
   gcttctcgtc cgttcgctcg ac 22
<210> 4
   <211> 24
   <212> DNA
   <213> artificial DNA
<220>
   <221> misc_binding
   <222> (1)..(24)
   <223> hybridization probe for the specific detection of Escherichia coli RNA
<400> 4
   actttactcc cttcctcccc gctg 24
<210> 5
   <211> 25
   <212> DNA
   <213> artificial DNA
<220>
   <221> misc_binding
   <222> (1)..(25)
   <223> hybridization probe for the specific detection of Salmonella enterica RNA
<400> 5
   aaatcacttc acctacgtgt cagcg 25

## Description

The present invention relates to a new composition for permeabilizing the walls of microorganisms, comprising the combination of ethylenediaminetetraacetic acid (EDTA) and polyethyleneimine (PEI), and to uses thereof, in particular in a method for the targeted counting and/or identification of microorganisms in a liquid or gaseous medium.

The invention applies more particularly to controlling the microbiological quality of liquid and gaseous media that enter, for example, into production lines for food and pharmaceutical products.

In this field, the microorganisms to be detected are often sparse since they are diluted in large volumes of water or of gas. However, their identity must be determined with certainty. In fact, the control carried out must make it possible to detect, in as short a period of time as possible, any contamination of the fluids that are part of the composition of a manufactured product, in order to be able to stop, where appropriate, the manufacture of the product or the distribution thereof, and to take the necessary decontamination steps.

Many methods for detecting microorganisms have been developed in order to minimize the culture time for said microorganisms.

Such a method for detecting microorganisms, comprising filtration of the liquid through a membrane, is described, for example, in application WO 01/59157. According to this method, the microorganisms contained in a liquid sample are retained at the surface of a membrane by passing the liquid through the latter. The microorganisms are cultured at the surface of the membrane in contact with an agar culture medium, for the period of time required to form a microcolony that is not visible to the naked eye. The cells forming the microcolony are then lysed in order to release their content of adenosine triphosphate (ATP) and of nucleic acids. The ATP released is used as a marker for identifying and quantifying the live cells by ATP-bioluminescence. The ATP serves as a substrate for an enzyme that produces a chemiluminescence reaction through which a light signal is obtained, and then detected using an appropriate video interface (for example: LCD camera). The signal obtained, provided in the form of an image, makes it possible to visualize in situ the site on the membrane where the microorganism has developed, in a manner similar to conventional counting carried out on a Petri dish in agar medium. In this case, the detection is said to be universal since ATP is a marker that is present in all living microorganisms. It does not make it possible to differentiate the various species of microorganisms.

The "Milliflex rapid^{®}" system, sold by the company Millipore, operates on the principle described above. It was designed to carry out the filtration and detection steps on one and the same membrane, which is held on a plastic support. This support is designed so as to slot into the various devices used for filtration, culturing the microorganisms, and detection.

This miniaturized system makes it possible to gain a notable amount of time compared with the conventional tests on Petri dishes. In addition, it makes it possible to store the images obtained on a digital medium, which is advantageous in terms of traceability of the products analyzed.

This system nevertheless has certain limits related to the fact that, in practice, the counting of the microorganisms present at the surface of the membrane is carried out after lysis of the cells. This lysis is made necessary in order to extract from the cells the products which react with the luminescent reagents, for example the ATP, to produce the luminescent signal that is detected. However, once the membrane has been treated, it becomes difficult to re-use it for the purpose of carrying out other analyses that may result, for example, in a precise identification of the microorganisms detected.

It is possible to determine the identity of microorganisms, for example, using probes that hybridize specifically to the nucleic acids (DNA or RNA) contained in the microorganisms. The most common probes of this type comprise an oligonucleotide fragment that is generally between 10 and 40 nucleotides in length, forming a sequence complementary to that within the DNA or the RNA of the microorganism.

Probes of PNA (peptide nucleic acid) type can also prove to be advantageous in this type of detection, since they have a peptide backbone that is less sensitive to the degrading enzymes present in the microorganisms and offer different labeling possibilities.

Application WO 2004/050902 describes a membrane detection system which makes it possible to detect the presence of microorganisms contaminating blood samples without causing any lysis of the microorganisms. The particularity of this system lies in the fact that the microorganisms are detected through the penetration of labeling agents into the microorganisms through their wall. The labeling agents used are molecules of small dimensions, in particular compounds that intercalate into nucleic acids (DNA, RNA), such as cyanin derivatives, propidium iodide, acridine orange or ethidium bromide. These small compounds penetrate relatively easily through the membrane into the microorganisms, using known permeabilizing agents of the prior art, such as polyethyleneimine (PEI), digitonin, nonensin, sodium hexametaphosphate or benzalkonium chloride. The liquid sample containing the microorganisms to be tested is first diluted in a permeabilizing solution comprising the labeling agent in the presence of a cell permeabilization reagent, and then the microorganisms are filtered through a membrane so as to be detected thereon. This results in a detection of the microorganisms that offers a better resolution.

However, due to the fact that the labels are universal labels, i.e. not specific for the microorganisms, this system does not make it possible to accurately determine the nature of the microorganisms detected.

In addition, in such a system, the manipulation of the microorganisms (incubation in the permeabilizing solution) takes place in solution, which requires specific precautions and complex equipment. It should be noted that the incubation of live cells in solution before filtration also has the drawback that the cells can continue to grow and to divide, before being fixed on the membrane, which creates, *de facto*, an uncertainty regarding the final result of the test.

Another difficulty linked to this type of analysis is the amount of permeabilizing reagents that must be used in the permeabilizing solution. This amount in fact varies according to the types of microorganisms to be detected. Depending, for example, on whether the microorganisms (in the case of bacteria) are of gram-positive or gram-negative type, the permeabilizing reagent will not have an effect at the same concentration, or worse, will cause lysis of the microorganisms present.

Thus, gram-positive bacteria have a single membrane that has a thick outer layer made up of peptidoglycans (a polymer made up of peptide and polysaccharide chains), thereby rendering their permeabilization more difficult than that of gram-negative bacteria, in which the wall consists of a double membrane between which the peptidoglycans are less abundant and more dispersed.

Polyethyleneimine (PEI), which is a weakly basic and polycationic aliphatic polymer, has been described as rendering the walls of certain gram-negative bacteria permeable to various antibiotic molecules [Helander, I.M. et al., Polyethyleneimine is an effective permeabilizer of gram-negative bacteria, Microbiology (1997), 143:3193-3199]. The concentration of PEI used for this purpose in order to obtain a bacterium such as *E. coli* is between 20 and 30 µg/ml.

Ethylenediaminetetraacetic acid (EDTA, of formula C₁₀H₁₆N₂O₈) is a well-known chelating agent that is commonly used in biochemistry as a stabilizing agent, in particular as an inhibitor of enzymatic or antioxidant reactions. EDTA is present in certain compositions, for this purpose. However, it has been demonstrated that EDTA taken alone does not allow satisfactory permeabilization of microorganism walls for the purpose of obtaining labeling of nucleic acids, in particular as regards gram-positive bacteria (WO 04/050902).

A lysozyme is a product that is used to permeabilize the walls of gram-positive bacteria in many protocols for transfecting cells with nucleic acids. It acts in particular by hydrolyzing certain bonds between the N-acetylglucosamine and N-acetylmuramic acids of the peptidoglycans present in the bacterial walls.

The aim of the present invention is to respond to the limitations of the existing detection systems mentioned above, in particular as regards gram-positive bacteria, that are reputed to be more difficult to treat.

Surprisingly, the inventors have noted that the combination of EDTA and PEI makes it possible to permeabilize microorganism walls more effectively than the compositions described in the prior art, in particular those containing only PEI or EDTA.

It has thus been possible to establish that compositions combining at least PEI and EDTA are particularly suitable for the permeabilization of microorganisms, in particular gram-positive bacteria.

These compositions make it possible in particular, according to the invention, to implement a method for labeling said microorganisms using oligonucleotide probes that can be used to specifically detect the microorganisms. This method comprises bringing a microorganism into contact with a composition according to the invention, comprising the combination of PEI and EDTA, in the presence of labeled macromolecules such as oligonucleotide probes or of PNA type, so that the latter can penetrate into the microorganisms through their wall in order to bind therein.

In addition, it appeared that the microorganisms thus treated tolerated high concentrations of PEI, greater than those normally used, without causing any marked lysis of the cells.

In the present invention, the term "microorganism" denotes any (eukaryotic or prokaryotic) live cell, unicellular organism, gamete or virus comprising a genetic inheritance contained in an envelope made up of a single or multiple wall.

A composition as defined above allows the penetration of macromolecules into the microorganisms, while at the same time limiting the destruction of their walls.

A macromolecule is defined as a molecule resulting from the association of a large number of simple molecules, such as polypeptides, proteins, glycoproteins, oligonucleotides, polysaccharides, nucleic acids (DNA, RNA), antibodies, or derivatives thereof. The macromolecules for which the invention is suitable are generally molecules of which the molecular weight is greater than 1000 daltons (1 kDa), preferably greater than 2000 daltons (2 kDa), and more preferably greater than 5000 daltons (5 kDa).

Polyethyleneimine (PEI) is a soluble product that is available in several forms. The polymerized monomeric form, which has a molecular mass of approximately 750 kDa, is a preferred form according to the invention (CAS No. 9002-98-6). PEI is used in many fields, in particular as an agent for the flocculation of proteins and nucleic acids in solution in various purification processes. It is also used as a permeabilizing agent due to the fact that it has the property of reversibly destructuring the phospholipids present in cell walls, thus rendering these walls permeable to agents which are not normally allowed to penetrate into the cells.

Thus, a first subject of the invention is a composition for permeabilizing the walls of microorganisms, comprising the combination of PEI and EDTA.

The concentration of PEI in the permeabilizing composition can be between 100 and 1000 µg/ml, preferably between 150 and 900 µg/ml, and more preferably between 200 and 800 µg/ml.

According to the invention, the EDTA is generally present in the permeabilizing composition at a final concentration of between 1 and 200 mM, preferably between 5 and 100 mM. According to a preferred aspect, the final EDTA concentration is greater than 50 mM, more preferably between 50mM and 100 mM.

Preferably, this composition also comprises lysozyme at a concentration of generally between 0.5 and 5 mg/ml, preferably between 1 and 4 mg/ml.

Another subject of the invention relates to the use of a composition according to the invention, and more particularly a method for detecting microorganisms on a membrane, comprising a step consisting of penetration of labeled macromolecules into the microorganisms.

For reasons which are not yet well known, the combination of PEI and EDTA facilitates the penetration of macromolecules, such as nucleotide probes, into microorganisms, while the use of lysozyme is useful for fragmenting the peptidoglycans present in the walls of said microorganisms.

Preferably, the composition according to the invention does not comprise any detergent, therefore providing several advantages from the point of view of the method for detecting microorganisms detailed hereinafter. The absence of detergent makes it possible to prevent, for example, the destruction of certain proteins required for the implementation of the method, such as the lysozyme and the enzymes that participate in the production of the signal for detection by luminescence. The absence of detergent also makes it possible to be able to carry out the fixing of the microorganisms on the membrane by crosslinking, more effectively.

The specific probes that participate in the detection of the microorganisms are preferably oligonucleotide probes that can hybridize to the ribosomal RNAs of said microorganisms. These probes preferably comprise one of the following sequences:
SEQ ID NO.1: 5'TCTACCGCGTCACTTACGTGACACC 3'
SEQ ID NO.2: 5'TTTGTGGGATTGGCTTAACCTCGCG 3'
SEQ ID NO.3: 5'GCTTCTCGTCCGTTCGCTCGAC 3'
SEQ ID NO.4: 5'ACTTTACTCCCTTCCTCCCCGCTG 3'
SEQ ID NO.5: 5'AAATCACTTCACCTACGTGTCAGCG 3'

These sequences allow the specific detection of *Pseudomonas aeruginosa, Bacillus subtilis, Staphylococcus aureus, Escherichia coli* and *Salmonella enterica arizonae*, respectively.

The macromolecules that can be used in the method for detecting microorganisms, according to the invention, are not limited to oligonucleotide probes and can consist, for example, of antigens, antibodies or aptamers directed against internal constituents of the microorganisms, which can also be used to detect the microorganisms according to the invention.

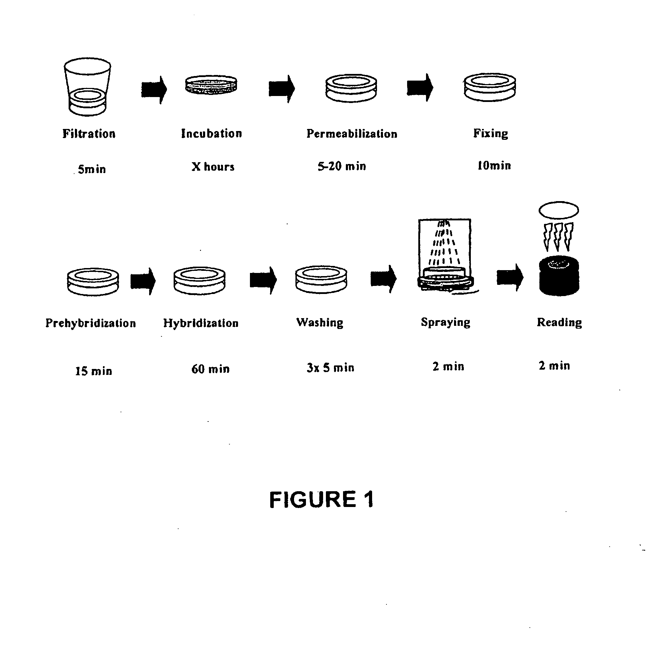

**Figure 1** illustrates the various steps of the method for specifically detecting microorganisms according to the invention as it is implemented in example 1.

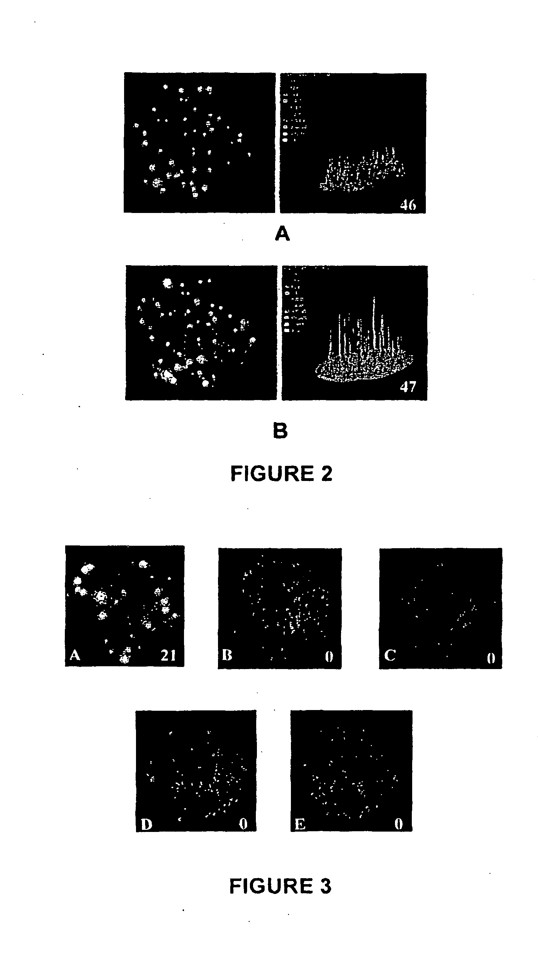

**Figure 2** compares **(A)** the results obtained according to the method of universal detection by ATP bioluminescence and **(B)** the results obtained using permeabilizing composition No. 7 comprising, as reagent, 1 mg/ml of lysozyme, 100 mM of EDTA, 374.5 µg/ml of PEI and an RNAse inhibitor (1x concentrated), for specifically detecting *B.subtilis* according to the method of the invention using a hybridization probe, the sequence of which corresponds to SEQ ID NO.2. The methods according to (A) and (B) are carried out on the same membrane, on which cells of the gram-positive bacterium *B.subtilis* are deposited. The left part shows, in a computer-generated image, the position of the microcolonies of bacteria detected by luminescence on the membrane, the right part represents the same membrane on an inclined plane with an indication in relief representing the intensity of the light signal emitted by each of the microcolonies detected. The number of microcolonies giving rise to a significant light signal is indicated in the bottom right of the computer-generated image. The number of microcolonies counted is virtually the same in (A) and (B), with, however, a signal that gives a better discrimination for (B).

**Figure 3** represents the computer-generated images obtained when a probe specific for *B.subtilis* is used according to the invention with respect to bacterial colonies of **(A)** *B. subtilis*, **(B)** *S. aureus*, **(C)** *P. aeruginosa*, **(D)** *E. coli*, and **(E)** *S. enterica subsp arizonae*. This figure shows that the method according to the invention makes it possible to obtain specific detection of the microorganisms.

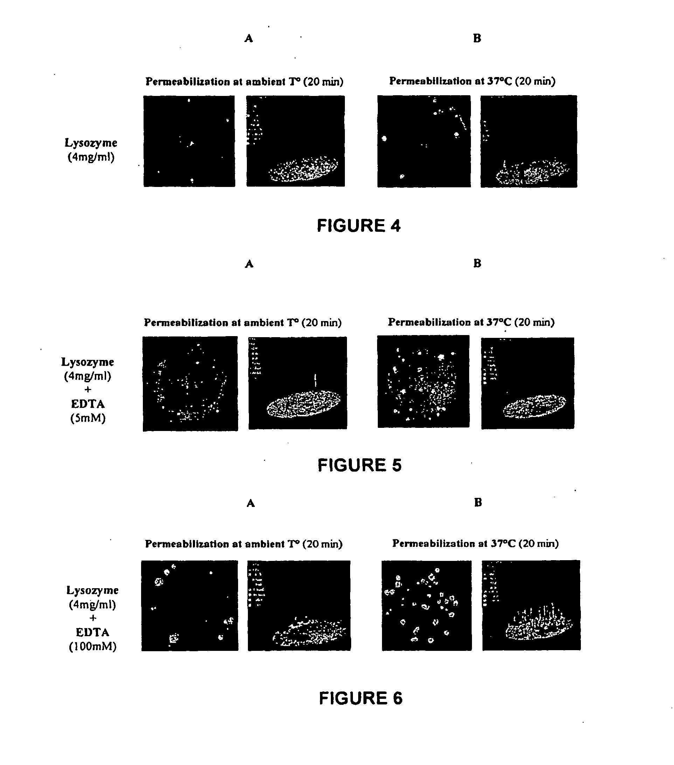

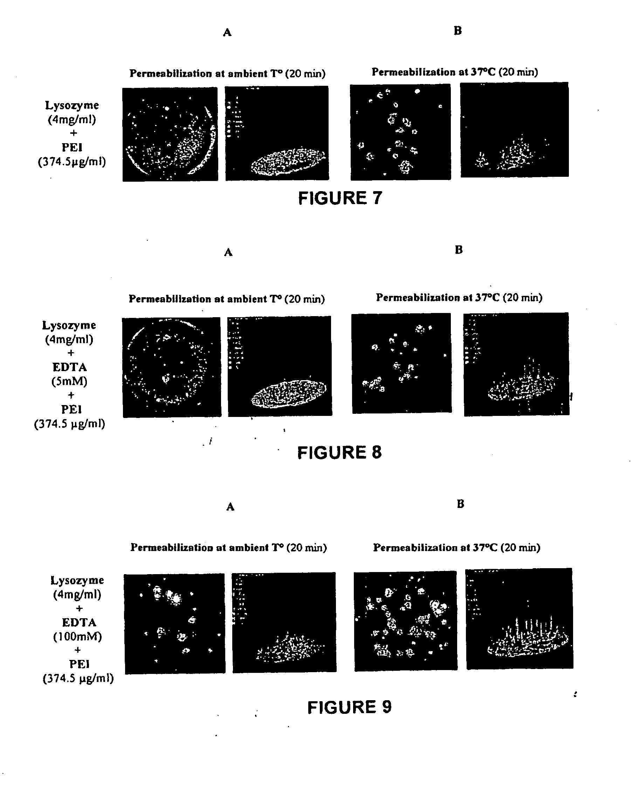

**Figures 4 to 9** represent the computer-generated images obtained according to the protocol described in example 1 using *B.subtilis* as target microorganism. Each figure corresponds to the use of a permeabilizing composition targeted in example 1. In these figures, **(A)** the permeabilization is carried out at ambient temperature, **(B)** the permeabilization is carried out at 37°C. The left part shows, as a computer-generated image, the position of the microcolonies of bacteria detected by luminescence on the membrane, the right part represents the same membrane on an inclined plane with an indication in relief representing the intensity of the light signal emitted by each of the microcolonies detected.

**Figure 4** corresponds to the use of a permeabilizing composition containing 4 mg/ml of lysozyme (composition No. 1) as permeabilizing reagent.

**Figure 5** corresponds to the use of a composition comprising 4 mg/ml of lysozyme and 5 mM of EDTA (composition No. 2) as permeabilizing reagent.

**Figure 6** corresponds to the use of a composition comprising 4 mg/ml of lysozyme and 100 mM of EDTA (composition No. 3) as permeabilizing reagent.

**Figure 7** corresponds to the use of a composition comprising 4 mg/ml of lysozyme and 374.5 µg/ml of PEI (composition No. 4) as permeabilizing reagent.

**Figure 8** corresponds to the use of a composition comprising 4 mg/ml of lysozyme, 5 mM of EDTA and 374.5 µg/ml of PEI (composition No. 5 according to the invention) as permeabilizing reagent.

**Figure 9** corresponds to the use of a composition comprising 4 mg/ml of lysozyme, 100 mM of EDTA and 374.5 µg/ml of PEI (composition No. 6 according to the invention) as permeabilizing reagent.

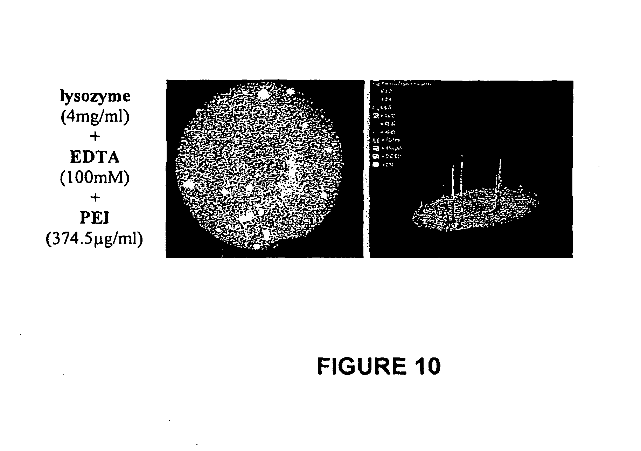

**Figure 10** represents the computer-generated image obtained when the gram-negative bacterium *Pseudomonas aeruginosa* is specifically detected according to the method of the invention using a permeabilizing composition comprising 4 mg/ml of lysozyme, 100 mM of EDTA and 374.5 µg/ml of PEI as reagent. The left part shows, as a computer-generated image, the position of the microcolonies of bacteria detected by luminescence on the membrane, the right part represents the same membrane on an inclined plane with an indication in relief representing the intensity of the light signal emitted by each of the microcolonies detected.

A subject of the invention is more particularly the use of the permeabilizing composition as described above, in a method for counting and/or identifying, on a membrane, the microorganisms initially present in a liquid or gaseous medium.

According to a specific embodiment of the invention, the method is **characterized in that** it comprises the following steps:
(a) the liquid or gaseous medium is filtered through a membrane such that the microorganisms present in this medium are retained on or in the membrane;
(b) the membrane and the microorganisms are brought into contact with a permeabilizing composition as described above, comprising PEI, EDTA and lysozyme;
(c) the cells are fixed on the membrane using a crosslinking agent;
(d) the microorganisms are brought into contact with one or more optionally labeled macromolecules capable of crossing the microorganism wall; and
(e) the macromolecules that have penetrated into the microorganisms are detected.

The microorganisms targeted by the method of the invention are more particularly chosen from gram-positive or gram-negative pathogenic bacteria of the genera *Pseudomonas, Escherichia, Legionella, Salmonella, Listeria, Bacillus, Streptococcus, Vibrio, Yersinia, Staphylococcus, Mycobacterium, Shigella, Clostridium, Campylobacter* or *Aeromonas*; protozoa of the genera *Giardia, Entamoeba, Cryptosporidium* or *Cyclospora*; mycoplasmas of the genera *Mycoplasma* and *Ureaplasma*, fungi of the genera *Aspergillus, Candida* or *Penicillium*, and hepatitis A to E viruses, which are microorganisms that are pathogenic in nature and are commonly encountered in the environment.

This method is, in principle, also applicable to the detection of unicellular algae such as cyanobacteria, to unicellular forms of parasitic organisms such as amebae or nematode, trematode or ascarid eggs, or else plant or animal gametes such as pollens or spermatozoa.

The aim of this method is to count the microorganisms present in a liquid medium such as water, or a gaseous medium such as air, while at the same time determining, as much as possible, their identity (family, genus, species, etc.). The term "liquid or gaseous medium" is intended to mean any fluid that can be filtered by applying a pressure difference through a membrane having pores with an average diameter of generally between 0.1 and 1.5 microns, preferably between 0.15 and 0.8 microns, more preferably between 0.2 and 0.6 microns. Such a fluid may consist of pure solutions involved in the manufacture of sterile products, but also of complex solutions such as drinking water, beverages, medical fluids (serum, urine, amniotic fluid, etc.).

The present method makes it possible, moreover, to have applications in the diagnostic field for the analysis of liquid samples originating from animals or from patients.

The term "membrane" denotes, in the present application, a synthetic support which has two faces, the pores of which have a known average diameter.

The membrane used in the context of the present invention has, in general, a high surface/volume ratio and a constant thickness of preferably between 90 and 200 microns.

Such a membrane may be monolayer or multilayer. In general, it consists of one or more materials chosen from polytetrafluoroethylene, polyvinylidene fluoride (PVDF), polycarbonate, polyamide, polyester, polyether sulfone, acetylcellulose and nitrocellulose.

The materials are preferably chosen so as to be compatible with the solvents used, in particular, the alcohols and aldehydes that may be used in the various steps of the method.

The membrane on which the microorganisms are detected preferably consists mainly of PVDF (polyvinylidene fluoride) or Nylon^{®}. More preferably, it is a PVDF filtering membrane, of the type of that sold by the company Millipore^{®} under the trademark Milliflex and the references MXHVWP124 or RMHVMFX24.

Step a) of the method according to the invention consists in filtering the liquid or gaseous medium to be analyzed through a membrane as defined above so as to retain, at the surface of this membrane, the microorganisms contained in said medium.

Once the microorganisms have been filtered and retained on the membrane, an optional step of culturing the microorganisms in contact with an appropriate culture medium can be included in the method, between steps a) and b). This culture medium is preferably an agar medium on which the membrane is placed after filtration. This step, which is optional, makes it possible to obtain colonies of each of the microorganisms initially filtered, in order to be able to detect them more readily..

According to the invention, the microorganisms are then incubated, in step b), in a permeabilizing composition according to the invention.

This incubation step is carried out in a small volume of solution that forms a film at the surface of the membrane. Advantageously, the permeabilizing composition can be contained in a solid support on which the membrane will be placed, such as, for example, an impregnated pad, thereby limiting any possible movement of liquid at the surface of the membrane, and therefore mixing of the microorganisms.

It is carried out at a temperature of between 20°C and 37°C for a period of from 5 to 20 minutes.

The method according to the invention is all the more effective since it comprises a step (c) via which the cells are fixed on the membrane using an agent that serves as a crosslinking agent.

This step makes it possible to fix the cells on the support that constitutes the membrane. A preferred crosslinking agent according to the invention is chosen from glutaraldehyde, formaldehyde and paraformaldehyde, paraformaldehyde being defined as a molecule composed of at least six units of formaldehyde. Advantageously, the fixing composition can be contained in a solid support on which the membrane will be placed, such as, for example, an impregnated pad, thereby limiting any possible movement of liquid at the surface of the membrane, and therefore mixing of the microorganisms. Preferably, according to the invention, the fixing step is carried out at a temperature of between 20°C and 35°C for a period of from 5 to 20 minutes.

This fixing step can also be carried out by irradiation of the microorganisms on the membrane using UV-radiation. The membrane then preferably consists of Nylon^{®}.

Step d) of bringing the microorganisms into contact with one or more optionally labeled macromolecules, which penetrate(s) through the wall of the microorganisms into said microorganisms, is preferably carried out at the end of the respective incubation and fixing steps b) and c), since it is at this precise moment that the microorganism walls were observed to be the most permeable to macromolecules.

According to a preferred embodiment of the invention, the macromolecules are labeled and used as probes.

The term "probe" is herein intended to mean macromolecules capable of recognizing and of associating with a biological element that is a constituent of the microorganism, thus allowing said microorganism to be labeled.

According to a preferred aspect of the invention, the probe is a macromolecule capable of hybridizing with DNA or RNA sequences present in the microorganism and having a certain degree of specificity with respect to said nucleic acids. The invention provides for any type of probe known to those skilled in the art that can give rise to hybridization with nucleic acids, such as, for example, simple oligonucleotides, oligonucleotides of 2'-O-methyl-RNA type, probes of PNA type (probes consisting of a polypeptide chain substituted with purine or pyrimidine bases) [Nielsen P.E. et al. Science (1991) 254:1497-1500] or probes of LNA type (oligonucleotides comprising one or more monomers of 2'-O-4'-C-methylene-β-D-ribofuranosyl) as described in patent EP 1 013 661.

The inventors have been able to note that the probes capable of hybridizing the ribosomal RNAs of microorganisms are particularly useful for carrying out the invention.

One aspect of the invention therefore consists of probes comprising a sequence identical to SEQ ID NO.1, SEQ ID NO.2, SEQ ID NO.3, SEQ ID NO.4 or SEQ ID NO.5, or else probes with a sequence exhibiting at least 80% identity, preferably 90%, more preferably 95%, with the sequences indicated above. Such probes are particularly indicated for the implementation of a method of counting and/or identification according to the invention. According to another aspect of the invention, the macromolecules that are made to penetrate into the microorganisms, and that may be of the same nature as the probes described above, are used as primers for carrying out an additional step of amplification specific for the nucleic acids contained in the microorganisms. If such macromolecules are used in the method according to the invention, then an additional, but nevertheless optional, step is introduced into the method between steps c) and e), preferably between steps d) and e).

Such an amplification step may prove to be useful for optimal detection of the microorganisms, by increasing the amount of nucleic acids available for the purposes of the detection. If this amplification step is highly specific, it also makes it possible to identify the microorganisms with greater selectivity and thus to decrease the number of false positives. The amplification is preferably an amplification of TMA or NASBA type [Compton, J., Nature (1991) 350:91-92] or of LAMP type [Notomi T., Nucleic Acids Research (2000), 28(12):e63].

The probes or primers described above can be designed so as to detect a single or several types of microorganisms. In this respect, they can have a variable degree of specificity with respect to the biological elements present in the microorganisms. For example, if the primers or probes are nucleic acids, chosen so as to hybridize with the nucleic acids whose sequence is conserved in many species of microorganisms, the identification will be relatively nonspecific; if, on the other hand, their sequence is chosen so as to recognize only sequences specific to a small number of species, the specificity will be greater.

According to yet another specific embodiment of the invention, and advantageously, a step of specific hybridization of the nucleic acid probes or of the primers with the nucleic acids of said microorganisms can be carried out after step d) and before the detection step e) that follows.

This step of hybridization of the nucleic acid probes or primers is carried out under standard hybridization conditions known to those skilled in the art. It can also comprise a washing step for eliminating the nucleic acid probes or primers that have produced nonspecific hybridizations.

For the purposes of the detection, the nucleic acid probes or primers according to the invention are preferably fluorescently labeled or else coupled to enzymes for carrying out a chemiluminescence reaction.

The microorganism detection step (e) is carried out by detecting the light signal originating from the chemiluminescence reaction or the emission of fluorescence obtained, corresponding to the labeling of the probe or of the primer, using an appropriate interface.

According to a more preferred aspect of the invention, the nucleic acid probes such as oligonucleotides are coupled to peroxidase. When peroxidase is placed in the presence of luminol (substrate for peroxidase), said peroxidase emits photons (bioluminescence reaction). The photons emitted by the microcolonies at the surface of the membrane can be captured through an optical fiber so as to reach a photocathode which converts them to electrons. Each electron will generate an electron cloud which, when reaching a phosphorus screen, is converted to photons. The CCD camera records the light emitted 30 times per second during the integration and the image is transmitted to the image analyzer.

According to yet another aspect, a permeabilizing composition as described above according to the invention can be used to carry out an *in vitro* diagnostic test. The specific properties of a composition according to the invention can in fact be used to cause nucleotide probes or any other molecular complex to penetrate into cells taken from humans or animals, or maintained in culture, in order to carry out the *in situ* detection.

The permeabilizating composition according to the invention can also find many applications in the medical field, for example as a drug adjuvant for increasing wall permeability to active ingredients such as antibiotics or antivirals that could not readily penetrate naturally into the cells.

More particularly, a composition according to the invention can be used to cause antisense nucleic acids, particularly antisense RNAs (iRNAs) to penetrate into cells in which one seeks to inhibit the expression of certain genes, in particular in the treatment of genetic diseases or of certain cancers.

The present invention is intended to also cover a kit for detecting and counting microorganisms in a liquid or gaseous medium, comprising at least:
- a membrane for filtering a liquid or gaseous medium; and
- a permeabilizing composition as described in the present invention.

Preferably, the membrane contained in this kit consists mainly of PVDF or of Nylon^{®}.

Advantageously, such a kit also comprises a composition comprising a crosslinking agent chosen from glutaraldehyde, formaldehyde and paraformaldehyde. This second composition makes it possible to fix the microorganisms on the membrane, thereby making it possible, by carrying out the method according to the invention, to obtain a better resolution of the detection.

A kit according to the invention can also comprise a probe for the specific detection of the microorganism being sought, in particular a probe as defined above comprising a sequence exhibiting at least 80% identity with SEQ ID NO.1, SEQ ID NO.2, SEQ ID NO.3, SEQ ID NO.4 or SEQ ID NO.5.

The aim of the following examples is to illustrate the invention without limiting it.

### Example 1

### Universal detection of bacterial strains according to the Milliflex rapid method

The strains tested are conserved in cryotubes (Dutscher, ref. 028049) frozen at -80°C, prepared from ATCC, DSMZ and NCTC referenced lyophilized strains.

To produce the cryotubes, a 100 ml flask of TS broth (Biomérieux, ref. 41146) is inoculated on the day the tubes are prepared, and incubated at 35-37°C. During growth, OD measurements at 600 nm are carried out at regular intervals using a spectrophotometer (Eppendorf, Biophotometer ref. 6131 000.012). The growth is stopped when the OD reaches 0.5. 600 µl of cryoprotector (Sigma, cell freezing with glycerol, ref. C6039) and 600 µl of culture are dispensed into each cryotube. The tubes are mixed and then placed at -80°C.

A count is performed on the broth and on a nonfrozen cryotube, by plating out dilutions of 10⁻⁵ and 10⁻⁶. These dilutions are prepared in flasks of 9 ml of peptone water (Biomérieux, ref. 42111). 100 µl of each dilution is plated out with a spreader on a TSA agar (Biomérieux, ref. 43011) incubated for 24 hours at 35°C. The process is repeated three times. The following day, a cryotube is thawed and counted in the same manner. An API gallery (Biomérieux) makes it possible to identify and confirm the purity of the cryotubes prepared. For all the tests, the bacterial strains are diluted in tubes of peptone water (Biomérieux, ref. 42111). A cascade dilution is prepared by adding 1 ml of the cryotube of the strain used to 9 ml of the tube of peptone water until the desired dilution is obtained.

The filtration is carried out using the Milliflex Plus Vacuum pump (Millipore, ref. MXP PLUS01) in Milliflex Rapid funnels with a 0.45 µm PVDF membrane with a hydrophobic grid pattern (Millipore, ref. RM HVMFX24). The bacteria are added to 50-100 ml of physiological saline containing 0.9% NaCl (B.Braun, ref 0066570 E).

The membranes are incubated on Milliflex Tryptic-soya agar (Millipore, ref. MXSM CTS48) or R2A (Millipore, ref. MXSM CRA 48) solid medium cassettes in an incubator at 32.5°C +/- 2.5°C.

After an incubation, the duration of which is dependent on the bacterium used, the lysis and bioluminescence reagents (Millipore, ref. MXRP BLRST) are applied to the membrane by means of the "Milliflex Rapid Autospray Station" (Millipore, ref. MXRP SPRKT), which distributes 35 µl of each reagent evenly at the surface of the membrane. The membranes are then read by means of the Milliflex Rapid system (Millipore, ref. MXRP KT230) according to the manufacturer's instructions.

The Milliflex Rapid system is a complex system which detects, amplifies and records, using a CCD camera, the light emitted during the bioluminescence reaction. It is composed of a detection tower with a CCD camera, an amplifier, an image analyzer and a software for collecting, processing and recording the data.

The analyzer subtracts the background noise from the emitted signal and generates an image which is transmitted to the computer. A representation of the membrane then appears on the screen and the system counts the colonies present on the membrane, as is represented in figures 2 to 10.

### Specific detection of bacterial strains according to the method of the invention using an oligonucleotide probe.

As for the Milliflex rapid system, the filtrations are carried out using the Milliflex Plus Vacuum pump in funnels with a 0.45 µm PVDF membrane (Millipore, ref. MX HVWP 124). The cells of the bacterial strains to be detected are added to 50-100 ml of physiological saline containing 0.9% NaCl. The membranes are incubated at 32.5°C +/- 2.5°C on Milliflex cassettes of TSA medium (Millipore, ref. MXSM CTS48), for the amount of time necessary for the microorganisms to form microcolonies.

The membranes are separated from the agar medium and placed in contact with 2 ml of permeabilizing solution on a leaktight support (absorbent pads of Millipore liquid media cassettes, ref. MXLM CO120) for 5 to 20 minutes at ambient temperature or at 37°C (depending on the nature of the microorganisms to be detected). The following permeabilizing solutions were tested:
Permeabilizing solution No. 1
   - lysozyme 4 mg/ml Permeabilizing solution No. 2
   - lysozyme 4 mg/ml
   - EDTA 5 mM Permeabilizing solution No. 3
   - lysozyme 4 mg/ml
   - EDTA 100 mM Permeabilizing solution No. 4
   - lysozyme 4 mg/ml
   - PEI 374.5 µg/ml Permeabilizing solution No. 5 (according to the invention)
   - lysozyme 4 mg/ml
   - EDTA 5 mM
   - PEI 374.5 µg/ml Permeabilizing solution No. 6 (according to the invention)
   - lysozyme 4 mg/ml
   - EDTA 100 mM
   - PEI 374.5 µg/ml

Once the membranes have been treated with the permeabilizing solution, they are brought into contact, for 10 minutes at ambient temperature, with 2 ml of a fixing solution comprising:
- H₂O₂ + urea 0.01%
- Formaldehyde 1%
- Ethanol 80%
- H₂O qs 1 L

The membranes are then transferred onto a hybridization cassette, so as to be brought into contact with 1.5 ml of a prehybridization solution comprising:
- Dextran 10%
- NaCl 1.74%
- Formamide 30%
- Sodium pyrophosphate 0.1%
- PVP 0.2%
- Ficoll 0.2%
- EDTA 0.186%
- Triton X-100 1%
- Tris-HCl 0.738%
- H₂O qs 1 L

They are then incubated at 50°C for 15 minutes with gentle agitation (10 rpm). The prehybridization solution is then replaced with 1.5 ml of the same solution comprising 100 mM of the probe, so as to form a film at the surface of the membrane.

The membrane is thus incubated in the hybridization solution at 50°C for 60 minutes with gentle agitation (10 rpm).

The membrane is then washed with 20 ml of a washing solution comprising (pH = 10):
- CAPSO 0.26%
- Tween 20 0.2%
- H₂O qs 1 L

This washing solution is diluted in 1 liter of water preheated to 50°C. The membrane is introduced into the washing solution with agitation (30 rpm), three times.

Specific hybridization cassettes and a specific washing tank are available under the references Millipore^{®} MXREXPEND and MXRPWASHR.

The membrane is then treated using a spray comprising the luminescence reagents that act on the peroxidase molecules coupled to the oligonucleotide probes (luminol and then hydrogen peroxide - Millipore, ref.
WBKL S0050), using a spraying station (Milliflex Rapid Autospray Station), and then analyzed by imaging according to the Milliflex rapid^{®} R-pseudo program in the same way as for the universal detection.

The various chemical products tested in the permeabilization assays come from the company Sigma-Aldrich^{®}: lysozyme (ref. L6876), EDTA (ref. E5134), polyethyleneimine (ref. P3143), chlorhexidine digluconate (ref. C9394), arginine ethyl ester (ref. A2883), proteinase K (ref. P4850), RNase inhibitor (ref. R7397). The solutions are prepared in PBS (Gibco, ref. 20012-019) and applied to the membranes using the liquid media cassettes.

The results obtained using the various permeabilizing solutions 1 to 6 mentioned above, for the purpose of specifically detecting the gram-positive bacterium *Bacillus subtilis*, are illustrated in figures 4 to 9.

In order to verify that all the bacteria present on the membrane have been effectively permeabilized, the ratio of the number of colonies detected according to the permeabilization and hybridization protocol (using an oligonucleotide probe of sequence SEQ ID NO.2) according to the invention, to the number of colonies detected by conventional counting on agar medium, was calculated (degree of coverage). The results obtained and the corresponding degree of coverage have been reported in table 1 below.

**Table 1:**

| Composition | % coverage (permeabilization amb. T°) | % coverage (permeabilization at 37°C) |
|---|---|---|
| Lysozyme (4 mg/ml) | 12.5% | 13.7% |
| Lysozyme (4 mg/ml) + EDTA (5 mM) | 18.8% | 13.7% |
| Lysozyme (4 mg/ml) + EDTA (100 mM) | 22.9% | 33.9% |
| Lysozyme (4 mg/ml) + PEI (374.5 µg/ml) | 25% | 47.9% |
| Lysozyme (4 mg/ml) + EDTA (5 mM) + PEI (374.5 µg/ml) | 22.9% | 39.5% |
| Lysozyme (4 mg/ml) + EDTA (100 mM) + PEI (374.5 µg/ml) | 33.9% | 60% |

It emerges from table 1 that the most effective permeabilizing solution is composition No. 6 comprising lysozyme (4 mg/ml), EDTA (100 mM) and PEI (374.5 µg/ml), in particular when it is used at 37°C. Subsequently, two concentrations of lysozyme were tested with regard to this composition No. 6 (1 and 4 mg/ml) in order to determine which one made it possible to obtain the best coverage. The comparison of these two concentrations was carried out according to the specific detection protocol described above, taking *B.subtilis* as detection target. The coverage results are presented in table 2.

**Table 2**

| | | |
|---|---|---|
| Lysozyme concentration | 1 mg/ml | 4 mg/ml |
| Coverage | 71.4% | 62.1% |

These results show that a lysozyme concentration of 1 mg/ml makes it possible to obtain better results according to the invention than a higher concentration of 4 mg/ml.

An "optimized" permeabilizing composition No. 7 comprising:
- Lysozyme 1 mg/ml
- EDTA 100 mM
- PEI 374.5 µg/ml
- RNAse inhibitor 1 X

was tested for *B.subtilis* according to the protocol targeted above, and, in parallel, according to the universal detection method. The result is represented in Figure 2. The coverage values calculated from the number of colonies counted by the R-Pseudo image analyzer are of the order of 100%.

Similar results were obtained with the gram-positive bacterium *Staphylococcus aureus* (Table 3) using an oligonucleotide probe of sequence SEQ ID NO.3.

**Table 3:**

| Number of CFU counted by the system after 6 h of incubation | Number of CFU counted by the system after 7 h of incubation | Number of CFU counted by the system after 8 h of incubation |
|---|---|---|
| 7 | 25 | 48 |
| 7 | 5 | 32 |
| 8 | 20 | 30 |
| 7 | 11 | 30 |
| 31 | 20 | 21 |
| Coverage | Coverage | Coverage |
| 52.2% | 73.9% | 139% |

Tests aimed at validating the specificity of the detection of *B. subtilis* according to the specific detection method described in the invention were carried out using 5 microorganisms: *B. subtilis, S. aureus, P. aeruginosa, E. coli* and *S*. *enterica subsp arizonae*. The test was carried out after overnight incubation at 25°C on TSA medium in order to allow growth of all the microorganisms. The result obtained using the probe specific for *B.subtilis* (SEQ ID NO.2) is represented in Figure 3.

Permeabilizing composition No. 6 (4 mg/ml Lysozyme + 100 mM EDTA + 374.5 µg/ml PEI) was used according to the invention on an impregnated pad for 20 minutes at 37°C for the purpose of specifically detecting the gram-negative bacterium *Pseudomonas aeruginosa* in the same way as for *B.subtilis.* To this effect, an oligonucleotide probe corresponding to the sequence SEQ ID NO.1 was used as hybridization probe. The image obtained at the end of this protocol, via the image analyzer, is represented in figure 10. It can be observed that the method according to the invention allows good discrimination of the bacterial microcolonies detected.

## Claims

1. Composition for permeabilizing the walls of microorganisms, **characterized in that** it comprises the combination of polyethyleneimine (PEI) and ethylenediaminetetraacetic acid (EDTA), the final concentration of PEI being greater than 100 µg/ml and the final EDTA concentration being greater than 50 mM in said composition.

2. Composition according to claim 1, **characterized in that** it further comprises lysozyme.

3. Composition according to either of claims 1 and 2, **characterized in that** the concentration of EDTA in the final composition is between greater than 50 and 150 mM, preferably between greater than 50 and 100 mM.

4. Composition according to any one of claims 1 to 3, **characterized in that** the concentration of PEI in said composition is between greater than 100 and 900 µg/ml, preferably between 200 and 800 µg/ml.

5. Composition according to any one of claims 1 to 4, **characterized in that** it does not comprise any detergent.

6. Method for counting and/or identifying, on a membrane, microorganisms initially present in a liquid or gaseous medium, **characterized in that** it comprises the following steps:
(a) the liquid or gaseous medium is filtered through a membrane such that the microorganisms present in this medium are retained on or in the membrane;
(b) the membrane and the microorganisms are brought into contact with a composition for permeabilizing walls of microorganisms according to claims 1 to 5;
(c) the cells are fixed on the membrane using a crosslinking agent;
(d) the microorganisms are brought into contact with one or more optionally labeled macromolecules capable of crossing the microorganism wall; and
(e) the macromolecules that have penetrated into the microorganisms are detected.

7. Method according to claim 6, **characterized in that** it comprises, between step a) and step b), an additional step of culturing the microorganisms.

8. Method according to either of claims 6 and 7, **characterized in that** the agent that serves as crosslinking agent in step c) is chosen from glutaraldehyde, formaldehyde and paraformaldehyde.

9. Method according to any one of claims 6 to 8, **characterized in that,** in step d), the macromolecule used is a hybridization probe, preferably an oligonucleotide or PNA-type hybridization probe.

10. Method according to claim 9, **characterized in that** the hybridization probe hybridizes to the RNAs of said microorganisms.

11. Method according to claim 10, **characterized in that** the hybridization probe hybridizes to the ribosomal RNA of said microorganisms.

12. Method according to any one of claims 9 to 11, **characterized in that** the hybridization probe used in step d) comprises an oligonucleotide sequence exhibiting at least 80% identity with a sequence chosen from SEQ ID NO. 1, 2, 3, 4 and 5.

13. Method according to any one of claims 6 to 12, **characterized in that,** in step d), the macromolecules are labeled by coupling with an enzyme that allows the emission of a light or fluorescent signal.

14. Method according to claim 13, **characterized in that** the detection of the microorganisms in step e) is carried out by means of chemiluminescence or fluorescence reaction and recognition of the light signal emitted using an appropriate interface.

15. Method according to any one of claims 6 to 14, **characterized in that** it comprises, between step d) and step e), an additional step of specific hybridization of the probes or primers with the nucleic acids of said microorganisms.

16. Method according to any one of claims 6 to 15, **characterized in that** it comprises, between step c) and step e), an additional step of specific amplification of the nucleic acids present in the microorganisms.

17. Method according to any one of claims 6 to 16, **characterized in that** the membrane on which the microorganisms are detected consists mainly of PVDF or nylon^{®}.

18. Use of a permeabilizing composition according to any one of claims 1 to 5, in order to cause macromolecules to penetrate into an isolated cell or microorganism.

19. Use of a permeabilizing composition according to any one of claims 1 to 5, in a method for counting and/or identifying microorganisms.

20. Kit for detecting and counting microorganisms, **characterized in that** it comprises:
- at least one labeled macromolecule; and
- a composition for permeabilizing the walls of microorganisms according to any one of claims 1 to 5.

21. Kit according to claim 20 , **characterized in that** it also comprises a composition comprising a crosslinking agent chosen from glutaraldehyde, formaldehyde and paraformaldehyde.

22. Kit according to any one of claims 20 or 21, **characterized in that** said labeled macromolecule consists of a hybridization probe.

23. Kit according to claim 22, **characterized in that** the labeled macromolecule consists of a hybridization probe comprising a sequence exhibiting at least 80% identity with one of the sequences SEQ ID NO. 1 to 5.

24. Kit according to any one of claims 20 to 23,
**characterized in that** it also comprises a filtering membrane, preferably a PVDF or Nylon^{®} membrane.

## Patentansprüche

1. Zusammensetzung zur Permeabilisierung der Wände von Mikroorganismen, **dadurch gekennzeichnet, dass** sie die Kombination von Polyethylenimin (PEI) und Ethylendiamintetraessigsäure (EDTA) umfasst, wobei die Endkonzentration von PEI größer als 100 µg/ml und die Endkonzentration von EDTA größer als 50 mM in der Zusammensetzung ist.

2. Zusammensetzung nach Anspruch 1, **dadurch gekennzeichnet, dass** sie ferner Lysozym umfasst.

3. Zusammensetzung nach einem der Ansprüche 1 und 2,
**dadurch gekennzeichnet, dass** die Konzentration von EDTA in der Endzusammensetzung zwischen größer als 50 und 150 mM, vorzugsweise zwischen größer als 50 und 100 mM beträgt.

4. Zusammensetzung nach einem der Ansprüche 1 bis 3, **dadurch gekennzeichnet, dass** die Konzentration von PEI in der Zusammensetzung zwischen größer als 100 und 900 µg/ml, vorzugsweise zwischen 200 und 800 µg/ml beträgt.

5. Zusammensetzung nach einem der Ansprüche 1 bis 4,
**dadurch gekennzeichnet, dass** sie kein Detergens umfasst.

6. Verfahren zum Zählen und/oder Identifizieren von Mikroorganismen, die ursprünglich in einem flüssigen oder gasförmigen Medium vorhanden sind, auf einer Membran, **dadurch gekennzeichnet, dass** das Verfahren die folgenden Stufen umfasst:
(a) das flüssige oder gasförmige Medium wird durch eine Membran derart filtriert, dass die in diesem Medium vorhandenen Mikroorganismen auf oder in der Membran zurückgehalten werden;
(b) die Membran und die Mikroorganismen werden mit einer Zusammensetzung zur Permeabilisierung von Wänden von Mikroorganismen gemäß den Ansprüchen 1 bis 5 in Kontakt gebracht;
(c) die Zellen werden auf der Membran unter Verwendung eines Vernetzungsmittels fixiert;
(d) die Mikroorganismen werden mit einem oder mehreren, optional markierten Makromolekülen, die die Mikroorganismenwand durchqueren können, in Kontakt gebracht; und
(e) die Makromoleküle, die in die Mikroorganismen eingedrungen sind, werden detektiert.

7. Verfahren nach Anspruch 6, **dadurch gekennzeichnet, dass** es zwischen Stufe a) und Stufe b) eine zusätzliche Stufe der Kultivierung der Mikroorganismen umfasst.

8. Verfahren nach einem der Ansprüche 6 und 7,
**dadurch gekennzeichnet, dass** das Mittel, das als Vernetzungsmittel in Stufe c) dient, aus Glutaraldehyd, Formaldehyd und Paraformaldehyd gewählt ist.

9. Verfahren nach einem der Ansprüche 6 bis 8,
**dadurch gekennzeichnet, dass** in Stufe d) das verwendete Makromolekül eine Hybridisierungssonde, vorzugsweise eine Oligonucleotid- oder PNA-Typ-Hybridisierungssonde ist.

10. Verfahren nach Anspruch 9, **dadurch gekennzeichnet, dass** die Hybridisierungssonde an die RNAs der Mikroorganismen hybridisiert.

11. Verfahren nach Anspruch 10, **dadurch gekennzeichnet, dass** die Hybridisierungssonde an die ribosomale RNA der Mikroorganismen hybridisiert.

12. Verfahren nach einem der Ansprüche 9 bis 11,
**dadurch gekennzeichnet, dass** die in Stufe d) verwendete Hybridisierungssonde eine Oligonucleotidsequenz umfasst, die mindestens 80% Identität mit einer Sequenz zeigt, die aus SEQ ID NO. 1, 2, 3, 4 und 5 ausgewählt ist.

13. Verfahren nach einem der Ansprüche 6 bis 12,
**dadurch gekennzeichnet, dass** in Stufe d) die Makromoleküle durch Kopplung mit einem Enzym, das die Emission eines Licht- oder Fluoreszenzsignals ermöglicht, markiert sind.

14. Verfahren nach Anspruch 13, **dadurch gekennzeichnet, dass** die Detektion der Mikroorganismen in Stufe e) mittels einer Chemilumineszenz- oder Fluoreszenzreaktion und der Erkennung des emittierten Lichtsignals unter Verwendung eines geeigneten Interface durchgeführt wird.

15. Verfahren nach einem der Ansprüche 6 bis 14,
**dadurch gekennzeichnet, dass** es zwischen Stufe d) und Stufe e) eine zusätzliche Stufe einer spezifischen Hybridisierung der Sonden oder Primer mit den Nucleinsäuren der Mikroorganismen umfasst.

16. Verfahren nach einem der Ansprüche 6 bis 15,
**dadurch gekennzeichnet, dass** es zwischen Stufe c) und Stufe e) eine zusätzliche Stufe einer spezifischen Amplifizierung der in den Mikroorganismen vorhandenen Nucleinsäuren umfasst.

17. Verfahren nach einem der Ansprüche 6 bis 16,
**dadurch gekennzeichnet, dass** die Membran, auf der die Mikroorganismen detektiert werden, hauptsächlich aus PVDF oder Nylon^{®} besteht.

18. Verwendung einer permeabilisierenden Zusammensetzung nach einem der Ansprüche 1 bis 5, um zu bewirken, dass Makromoleküle in eine isolierte Zelle oder einen isolierten Mikroorganismus eindringen.

19. Verwendung einer permeabilisierenden Zusammensetzung nach einem der Ansprüche 1 bis 5 in einem Verfahren zum Zählen und/oder Identifizieren von Mikroorganismen.

20. Kit zum Detektieren und Zählen von Mikroorganismen, **dadurch gekennzeichnet, dass** es umfasst:
- mindestens ein markiertes Makromolekül; und
- eine Zusammensetzung zur Permeabilisierung der Wände von Mikroorganismen gemäß einem der Ansprüche 1 bis 5.

21. Kit nach Anspruch 20, **dadurch gekennzeichnet, dass** es ferner eine Zusammensetzung umfasst, die ein aus Glutaraldehyd, Formaldehyd und Paraformaldehyd ausgewähltes Vernetzungsmittel umfasst.

22. Kit nach einem der Ansprüche 20 oder 21, **dadurch gekennzeichnet, dass** das markierte Makromolekül aus einer Hybridisierungssonde besteht.

23. Kit nach Anspruch 22, **dadurch gekennzeichnet, dass** das markierte Makromolekül aus einer Hybridisierungssonde besteht, die eine Sequenz umfasst, die mindestens 80% Identität mit einer der Sequenzen SEQ ID NO. 1 bis 5 zeigt.

24. Kit nach einem der Ansprüche 20 bis 23, **dadurch gekennzeichnet, dass** es ferner eine Filtriermembran, vorzugsweise eine PVDF- oder Nylon^{®}-Membran, umfasst.

## Revendications

1. Composition pour la perméabilisation des parois de microorganismes, **caractérisée en ce qu'**elle comprend l'association de polyéthylèneimine (PEI) et d'acide éthylène-diaminetétraacétique (EDTA), la concentration finale de PEI étant supérieure à 100 µg/ml et la concentration finale d'EDTA étant supérieure à 50 mM dans ladite composition.

2. Composition suivant la revendication 1, **caractérisée en ce qu'**elle comprend en outre du lysozyme.

3. Composition suivant l'une des revendications 1 et 2, **caractérisée en ce que** la concentration d'EDTA dans la composition finale est comprise entre plus de 50 et 150 mM, de préférence entre plus de 50 à 100 mM.

4. Composition suivant l'une quelconque des revendications 1 à 3, **caractérisée en ce que** la concentration de PEI dans ladite composition est comprise entre plus de 100 et 900 µg/ml, de préférence entre 200 et 800 µg/ml.

5. Composition suivant l'une quelconque des revendications 1 à 4, **caractérisée en ce qu'**elle ne comprend aucun détergent.

6. Procédé pour dénombrer et/ou identifier, sur une membrane, des microorganismes initialement présents dans un milieu liquide ou gazeux, **caractérisé en ce qu'**il comprend les étapes suivantes :
(a) le liquide ou milieu gazeux est filtré à travers une membrane de telle sorte que les microorganismes présents dans ce milieu soient retenus sur ou dans la membrane ;
(b) la membrane et les microorganismes sont mis en contact avec une composition de perméabilisation des parois de microorganismes suivant les revendications 1 à 5 ;
(c) les cellules sont fixées sur la membrane en utilisant un agent de réticulation ;
(d) les microorganismes sont mis en contact avec une ou plusieurs macromolécules facultativement marquées capables de traverser la paroi des microorganismes ; et
(e) les macromolécules qui ont pénétré dans les microorganismes sont détectées.

7. Procédé suivant la revendication 6, **caractérisé en ce qu'**il comprend, entre l'étape a) et l'étape b), une étape supplémentaire de culture des microorganismes.

8. Procédé suivant une des revendications 6 et 7, **caractérisé en ce que** l'agent qui sert d'agent de réticulation dans l'étape c) est choisi entre le glutaraldéhyde, le formaldéhyde et le paraformaldéhyde.

9. Procédé suivant l'une quelconque des revendications 6 à 8, **caractérisé en ce que,** dans l'étape d), la macromolécule utilisée est une sonde d'hybridation, de préférence une sonde oligonucléotidique ou une sonde d'hybridation du type PNA.

10. Procédé suivant la revendication 9, **caractérisé en ce que** la sonde d'hybridation s'hybride aux ARN desdits microorganismes.

11. Procédé suivant la revendication 10, **caractérisé en ce que** la sonde d'hybridation s'hybride à l'ARN ribosomal desdits microorganismes.

12. Procédé suivant l'une quelconque des revendications 9 à 11, **caractérisé en ce que** la sonde d'hybridation utilisée dans l'étape d) comprend une séquence oligonucléotidique présentant une identité d'au moins 80 % avec une séquence choisie entre les SEQ ID N° 1, 2, 3, 4 et 5.

13. Procédé suivant l'une quelconque des revendications 6 à 12, **caractérisé en ce que**, dans l'étape d), les macromolécules sont marquées par couplage avec une enzyme qui permet l'émission d'un signal lumineux ou fluorescent.

14. Procédé suivant la revendication 13, **caractérisé en ce que** la détection des microorganismes dans l'étape e) est effectuée au moyen d'une réaction de chimioluminescence ou de fluorescence et de la reconnaissance du signal lumineux émis en utilisant une interface appropriée.

15. Procédé suivant l'une quelconque des revendications 6 à 14, **caractérisé en ce qu'**il comprend, entre l'étape d) et l'étape e), une étape supplémentaire d'hybridation spécifique des sondes ou amorces avec les acides nucléiques desdits microorganismes.

16. Procédé suivant l'une quelconque des revendications 6 à 15, **caractérisé en ce qu'**il comprend, entre l'étape c) et l'étape e), une étape supplémentaire d'amplification spécifique des acides nucléiques présents dans les micro-organismes.

17. Procédé suivant l'une quelconque des revendications 6 à 16, **caractérisé en ce que** la membrane sur laquelle les microorganismes sont détectés consiste principalement en PVDF ou Nylon^{®}.

18. Utilisation d'une composition de perméabilisation suivant l'une quelconque des revendications 1 à 5, afin de provoquer la pénétration de macromolécules dans une cellule isolée ou un microorganisme.

19. Utilisation d'une composition de perméabilisation suivant l'une quelconque des revendications 1 à 5, dans un procédé de dénombrement et/ou d'identification de microorganismes.

20. Kit pour la détection et le dénombrement de microorganismes, **caractérisé en ce qu'**il comprend :
- au moins une macromolécule marquée ; et
- une composition de perméabilisation des parois de microorganismes suivant l'une quelconque des revendications 1 à 5.

21. Kit suivant la revendication 20, **caractérisé en ce qu'**il comprend également une composition comprenant un agent de réticulation choisi entre le glutaraldéhyde, le formaldéhyde et le paraformaldéhyde.

22. Kit suivant l'une quelconque des revendications 20 et 21, **caractérisé en ce que** ladite macromolécule marquée consiste en une sonde d'hybridation.

23. Kit suivant la revendication 22, **caractérisé en ce que** la macromolécule marquée consiste en une sonde d'hybridation comprenant une séquence présentant une identité d'au moins 80 % avec l'une des séquences SEQ ID N° 1 à 5.

24. Kit suivant l'une quelconque des revendications 20 à 23, **caractérisé en ce qu'**il comprend également une membrane de filtration, de préférence une membrane de PVDF ou de Nylon^{®}.
